(19) Europäisches Patentamt
European Patent Office
Office européen des brevets

(11) Veröffentlichungsnummer: **0 123 323**
**B1**

(12) # EUROPÄISCHE PATENTSCHRIFT

(45) Veröffentlichungstag der Patentschrift: **23.08.89**

(51) Int. Cl.⁴: **A 61 F 2/02**, C 08 L 33/14, A 61 L 25/00

(21) Anmeldenummer: **84104710.3**

(22) Anmeldetag: **26.04.84**

(54) **Verwendung von difunktionellen Acrylsäure- und Methacrylsäureestern zur Herstellung von härtbaren Knochenzementen.**

(30) Priorität: **26.04.83 DE 3314977**

(43) Veröffentlichungstag der Anmeldung:
**31.10.84 Patentblatt 84/44**

(45) Bekanntmachung des Hinweises auf die Patenterteilung:
**23.08.89 Patentblatt 89/34**

(84) Benannte Vertragsstaaten:
**AT BE CH DE FR GB IT LI LU NL SE**

(56) Entgegenhaltungen:
**EP-A-0 017 937**
**EP-A-0 023 013**
**DE-A-1 921 869**
**DE-A-2 656 847**

**Die Akte enthält technische Angaben, die nach dem Eingang der Anmeldung eingereicht wurden und die nicht in dieser Patentschrift enthalten sind.**

(73) Patentinhaber: **ESPE Stiftung & Co Produktions- und Vertriebs KG, D-8031 Seefeld (DE)**

(72) Erfinder: **Schmitt, Werner, Dr., Prinzenweg 10, D-8130 Starnberg (DE)**
Erfinder: **Gasser, Oswald, Dr., Hartstrasse 13, D-8031 Seefeld (DE)**

(74) Vertreter: **Müller, Bernhard, Dr., Graf-Toerring-Strasse 45, D-8031 Seefeld 2 (DE)**

**EP 0 123 323 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft die Verwendung spezieller, difunktioneller Acrylsäure- und Methacrylsäureester in härtbaren Knochenzementen zur Zementierung von Knochen. Die Knochenzemente bestehen üblicherweise aus zwei zu vermischenden Komponenten und können zur Befestigung von künstlichen Gelenken, zur Ausfüllung von Knochendefekten oder zur Anheftung von Kochenteilen untereinander verwendet werden.

Bisher wurden als Knochenzemente üblicherweise Massen verwendet, die durch Vermischen eines Pulvers mit einer Flüssigkeit und anschliessende Quellung erhalten werden. Bei den Pulvern handelt es sich um Polymerisate oder Copolymerisate von Acrylsäureestern oder Methacrylsäureestern, bevorzugt um Copolymerisate aus Methylmethacrylat und Methylacrylat in Form von feinen Perlen mit einer typischen Teilchengröße von etwa 50 µm. Diesem Polymerisat sind Röntgenkontrastmittel, z. B. Bariumsulfat oder Zirkondioxid, sowie meist ein Antibiotikum zugesetzt. Außerdem enthält das Pulver ein Peroxid als Polymerisationsinitiator.

Als Flüssigkeit wird üblicherweise Methylmethacrylat verwendet, das zumeist mit Farbstoff versetzt ist und als Polymerisationsbeschleuniger ein aromatisches Amin, z. B. N,N-Dimethyl-p-toluidin enthält. Ein derartiger Knochenzement ist z. B. in der DE-OS-2 511 122 beschrieben.

Nachteilig an diesen Knochenzementen ist ihre hohe Temperaturentwicklung bei der Erhärtung sowie die relativ hohe Toxizität des Methylmethacrylats, das in den Kreislauf gelangt und teilweise ein dramatisches Absinken des Blutdrucks bewirkt. Durch die hohen Temperaturen können Schädigungen des umgebenden Gewebes auftreten, so daß es zu einer Lockerung der Verbindung zwischen Zement und Knochen kommen kann. Durch die kombinierte toxische und thermische Einwirkung haben sich auch bereits Fettembolien ergeben. Auch die Festigkeit dieser Knochenzemente war in manchen Anwendungsfällen, z. B. bei der Befestigung von Hüftprothesen, nicht ausreichend, so daß sich Lockerungen der Implantate ergaben.

Es wurden zahlreiche Versuche unternommen, die vorstehend geschilderten Nachteile von herkömmlichen Knochenzementen auf Methylmethacrylatbasis zu beseitigen.

Gemäß der DE-OS-2 552 70 wird zur Vermeidung des Temperaturanstiegs das monomere Methylmethacrylat in einer wässrigen Emulsion eingesetzt. In bezug auf Festigkeit und Toxizität ergibt sich jedoch bei diesem Zement keinerlei Verbesserung.

Zur Verbesserung der mechanischen Eigenschaften wurden, wie in der DE-OS-2 724 814 beschrieben, anorganische Füllstoffe zusätzlich zu den polymeren Methacrylaten zugesetzt. Bei der Aushärtung dieser Zemente bleiben aber die Schwierigkeiten hinsichtlich der Wärmeentwicklung und der Toxizität unverändert bestehen. Auch die Zumischung von Apatit oder Kohlefasern, die in der EP-A-0 006 414 beschrieben ist, führt zu ähnlichen Ergebnissen. Auch die Bewehrung mit schwammartigen Elementen gemäß der DE-OS-3 042 003 führt nur zu einer Verbesserung der mechanischen Festigkeit, ohne daß die übrigen genannten Schwierigkeiten beseitigt werden.

Gemäß der DE-PS-2 229 702 wurde versucht, einen Teil des monomeren Methylmethacrylats durch monomeres Isobutylmethacrylat zu ersetzen. Dies brachte jedoch nur eine Erniedrigung der Temperaturspitze, ohne daß die toxikologischen und mechanischen Probleme zufriedenstellend gelöst wurden.

Auch die sogenannten niedrigviskosen Knochenzemente der DE-OS-3 106 452 bewirken nur eine bessere Verankerung des Knochenzements in den Unregelmässigkeiten des Knochens. Die Probleme aufgrund der Wärmeentwicklung und der toxischen Wirkung werden durch die Lehre dieser Druckschrift nicht beseitigt. In der EP-A-0 037 759 ist der Einsatz von Epoxiden zusammen mit den Hydroxylgruppen enthaltenden Polybutadienen als härtbare Komponente für einen Knochenzement beschrieben. Dieser Zement ist jedoch in der Anwendung unpraktisch, da er aus 4 Komponenten angemischt werden muß. Außerdem dauert die Aushärtung dieser Zemente relativ lange, obwohl als Beschleuniger Mercapto-2-ethanol eingesetzt ist. Hinzu kommt die gravierende Geruchsbelästigung durch das Mercapto-2-ethanol.

In der Europäischen Veröffentlichungsschrift 0 017 937 werden haftende Zusammensetzungen für harte menschliche Gewebe beschrieben, die besondere Haftungseigenschaften auch an feuchten Oberflächen von Zahnmaterialien besitzen sollen. Die Massen sollen sich auch zur Herstellung von Knochenzementen eignen. Die dort verwendeten Monomere müssen zwingenderweise wenigstens eine Carboxyl-, Epoxy-amino- oder Hydroxyl-Gruppe enthalten sowie mindestens 0,5 Gew.-% eines Metallalkoholats. Mit den Massen dieser Veröffentlichungsschrift kann eine bessere Verankerung des Knochenzements wohl erreicht werden, die Probleme aufgrund der Wärmeentwicklung und der toxischen Wirkung werden durch die Lehre dieser Druckschrift nicht beseitigt, zudem weisen die gefertigten Massen ungenügende Fliesseigenschaften, Aushärtungsgeschwindigkeiten und Festigkeiten auf.

Aufgabe der Erfindung ist es, einen gut verarbeitbaren Knochenzement bereitzustellen, der bei der Aushärtung eine geringe Temperaturerhöhung aufweist, sich durch geringe Toxizität und hohe Festigkeit der Polymerisate auszeichnet und rasch aushärtet.

Erfindungsgemäß wurde festgestellt, daß Knochenzemente, die den vorgenannten Anforderungen entsprechen, erhalten werden können, wenn man als polymerisierbare Komponente dieser Knochenzemente difunktionelle Acrylsäure- oder Methacrylsäureester der folgenden allgemeinen Formel

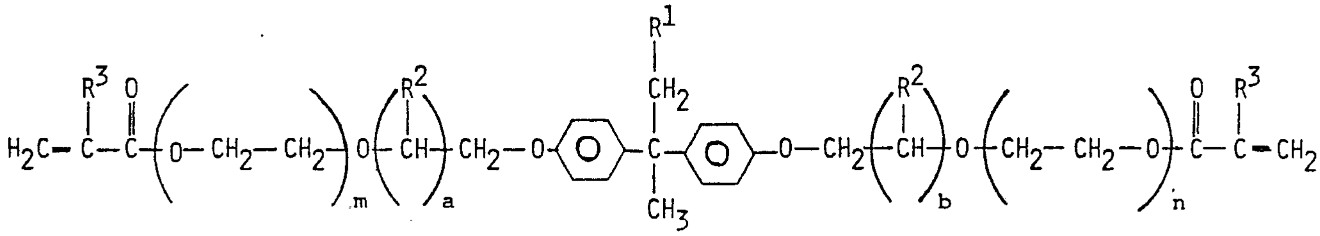

einsetzt, in der

a den Wert 1 oder 2 hat,
b den Wert 1 oder 2 hat,
m den Wert 0, 1 oder 2 hat,
n den Wert 0, 1 oder 2 hat,
$R^1$ H oder $OH_3$ bedeutet,
$R^2$ H, $OH_3$ oder $C_2H_5$ bedeutet und
$R^3$ H oder $CH_3$ bedeutet.

Verbindungen dieser Art und ihre Verwendung auf dem Dentalgebiet sind in der DE-PS-1 921 869 und in der DE-AS-2 656 847 beschrieben. Aufgrund der im Vergleich zum Dentalgebiet völlig unterschiedlichen Problemstellung konnte aber nicht erwartet werden, daß aus der Fülle der Acrylsäure- und Methacrylsäureester, die auf dentalem Gebiet Anwendung gefunden haben, gerade die erfindungsgemäßen Substanzen besonders geeignete Knochenzemente ergeben würden. Unter anderem treten auf dem Gebiet der Knochenzemente folgende Besonderheiten auf, die bei dental zu verwendenden Massen nur von untergeordneter Bedeutung sind. Bei Knochenzementen spielt die Toxizität der Einzelbestandteile eine besonders wichtige Rolle, da deren unmittelbare Aufnahme in das umliegende Gewebe und in den Kreislauf zu erwarten ist, während bei Dentalmassen eine Resorption der Bestandteile nur in Ausnahmefällen zu erwarten ist und durch Anwendung einer sogenannten Unterfüllung unterdrückt werden kann. Außerdem spielt bei der Füllung von Zähnen die Temperaturentwicklung der Massen beim Aushärten nur eine geringe Rolle, da die angewandten Mengen relativ klein sind und das Zahnmaterial relativ temperaturbeständig ist und eine gute und rasche Temperaturableitung gewährleistet ist. Naturgemäß ist bei Knochen, die ringsum bedeckt sind, ein wesentlich langsamerer Temperaturausgleich zu erwarten, auch sind die verwendeten Mengen vergleichsweise groß; außerdem besteht die Gefahr, daß durch erhöhte Temperaturen umliegendes Gewebe geschädigt und Proteine und dergleichen denaturiert werden.

Schließlich unterscheiden sich Zähne und Knochen auch hinsichtlich ihres Aufbaus, ihrer mechanischen Eigenschaften und ihrer regelmäßigen mechanischen Beanspruchung so weitgehend, daß aus der Eignung bestimmter Materialien als dentale Füllstoffe keine Rückschlüsse dahingehend gezogen werden können, ob sich derartige Substanzen auch als Knochenzemente eignen. Hierfür spricht auch die Tatsache, daß von den bisher für Dentalzwecke eingesetzten Massen sich keines der Produkte als zufriedenstellender Knochenzement erwiesen hat. Die erfindungsgemäß verwendeten Methacrylsäureester und Dimethacrylsäureester können nach den in der DE-PS-1 921 869 und in der DE-AS-2 656 847 beschriebenen Verfahren hergestellt werden.

Kristalline Vertreter der erfindungsgemäß verwendeten Substanzklassen werden, soweit sie Schmelzpunkte unter etwa 40°C aufweisen, durch Erwärmen in eine für die Applikation geeignete flüssige Form gebracht. Vorzugsweise werden sie jedoch in einem flüssigen Vertreter der erfindungsgemäßen Substanzklasse oder in einem anderen, flüssigen, physiologisch unbedenklichen Medium gelöst und zur Anwendung gebracht.

Generell können die erfindungsgemäß verwendeten Substanzen zusammen mit anderen, physiologisch unbedenklichen Acrylsäureestern oder Meth-Acrylsäureestern verwendet werden. Bevorzugt sind hierbei höhermolekulare, insbesondere difunktionelle acrylsäure- bzw. Methacrylsäureester, wie Dekan-di(meth)-acrylat oder Bis-hydroxymethyl-tricyclo-[5.2.1.$0^{2,6}$]decan-di(meth)acrylat. Derartige Zusätze können z. B. als Verdünner erwünscht sein.

Vorzugsweise werden jedoch die genannten Substanzen alleine eingesetzt, insbesondere die Verbindungen, die in flüssiger Form vorliegen. Aufgrund ihrer geringen Toxizität sind insbesondere die Methacrylate bevorzugt. Ein besonders bevorzugtes Produkt ist 2,2-Bis-p-(γ-hydroxypropoxy)-phenyl]-propan-dimethacrylat, das bei Raumtemperatur flüssig ist. Diese Verbindung kann gemäß Beispiel 10 der DE-PS-1 921 869 hergestellt werden.

Die erfindungsgemäß verwendeten Acrylsäure- und Methacrylsäureester können übliche Antioxidantien, Stabilisatoren, Weichmacher und/oder Pigmente enthalten.

Die Härtung kann mit allen üblichen Polymerisationsinitiatoren erfolgen. Naturgemäß kommen bei Knochenzementen nur die Methoden der Kalthärtung in Frage. Bevorzugt wird das Initiatorsystem Peroxid /aromatisches Amin. Geeignete Peroxide sind z. B. Dibenzoylperoxid, Bis-(p-chlor-benzoyl)-peroxid oder Bis-(p-tert.-butylbenzoyl)peroxid. Geeignete Amine sind z. B. N,N-Dimethyl-p-toluidin oder die in der DE-OS-2 658 58 beschriebenen tert.-Butyl-substituierten Aniline.

Weitere geeignete Initiatorsysteme sind die Kombinationen aus Barbitursäure/Peroxid/Kupferverbindung

/Halogenidion, die in der DE-AS-1 495 520 beschrieben sind, und die Kombinationen Malonylsulfamid/Peroxid /Halogenidion/Kupferverbindung, die in der DE-OS-3 107 577 erläutert sind. Insbesondere führt die Verwendung der Malonylsulfamide als Polymerisationsinitiator zu besonders niedrigen Temperaturspitzen während des Aushärtungsvorgangs.

Die Initiatorbestandteile werden in den üblichen Konzentrationen eingesetzt, die so gewählt werden müssen, daß dem Arzt genügend Verarbeitungszeit verbleibt, um den Knochenzement in die gewünschte Situation zu bringen. Andererseits muß anschließend eine relativ rasche Erhärtung gewährleistet sein, um eine möglichst kurze Dauer des chirurgischen Eingriffs zu ermöglichen.

Zur Erhöhung der mechanischen Festigkeit, zur Verringerung des Polymerisationsschrumpfes, zur weiteren Erniedrigung der Temperaturspitze und zur Erzielung einer für die Verarbeitung günstigen Konsistenz wird der erfindungsgemäße Knochenzement üblicherweise zusammen mit einem Füllstoff verwendet. Die Füllstoffmenge kann 10 bis 80 und vorzugsweise 20 bis 70 Gewichtsprozent betragen.

Es können alle üblichen organischen Füllstoffe eingesetzt werden, wie Poly(meth)acrylate oder Polyamide. Gut bewährt haben sich Polymethylmethacrylat und Copolymerisate aus Methylmethacrylat und Methylacrylat.

Auch anorganische Füllstoffe sind gut geeignet. Es kommen vor allem Kieselsäure, Silicatgläser, Keramik, Quarz und auch Mineralien, wie Apatit, Calciumfluorid und dergleichen, in Frage. Auch Kohlefasern und andere physiologisch unbedenkliche faserförmige verstärkende Füllstoffe können eingesetzt werden. Im Falle von Silicatfüllstoffen ist es zweckmäßig, diese zur Einbindung in die Acrylat- bzw. Methacrylatmatrix einer Oberflächenbehandlung zu unterziehen. Geeignet ist dabei vor allem die bekannte Behandlung mit Silanen, vorzugsweise mit γ-Methacroyl-oxypropyl-trimethoxy-silan.

Um dem Knochenzement Röntgenopazität zu verleihen, werden zumindest teilweise röntgensichtbare Füllstoffe oder Zusätze verwendet. Als röntgenopake Substanzen kommen barium-, strontium-, oder lanthanhaltige Gläser in Frage, aber auch Bariumsulfat, Lanthanoxid, Wismutphosphat, Calciumwolframat oder andere bekannte röntgenpositive Zusätze. Prinzipiell kann die Röntgenopazität auch durch Einführung von schweren Atomen, z. B. Brom- oder Jodatomen, in eines der polymerisierbaren Monomeren oder in einen polymeren Zusatz erzielt werden.

Zur Kontrolle der Viskosität und zur Verhinderung des Absetzens der Füllstoffe hat es sich bewährt, mikrofeine Kieselsäure, die ebenfalls oberflächlich mit Silan behandelt werden kann, dem efindungsgemäßen Knochenzement zuzusetzen. Die mikrofeine Kieselsäure kann jedoch auch in größerer Menge direkt als Füllstoff eingesetzt werden.

Zur Kontrolle der Viskosität und zur Beeinflussung des Elastizitätsverhaltens des ausgehärteten Knochenzements kann es weiterhin vorteilhaft sein, den Monomeren lösliche Polymere zuzusetzen.

Üblicherweise werden die erfindungsgemäßen Knochenzemente in zwei Komponenten dargeboten, auf die die aktiven Komponenten des Initiatorsystems so verteilt sind, daß eine gute Lagerfähigkeit dieser getrennten Bestandteile erzielt wird.

So kann z. B. das Peroxid unter den Füllstoff gemischt werden, während das Amin in der die erfindungsgemäßen polymerisierbaren Monomeren enthaltenden Monomermischung gelöst ist. Der Knochenzement wird durch Vermischen von Flüssigkeit mit Feststoff kurz vor der Anwendung zubereitet.

In vorteilhafter Weise, besonders bei der Verwendung von anorganischen Füllstoffen, kann der Knochenzement auch in Form von 2 Pasten zur Verfügung gestellt werden. Die erste Paste besteht dabei aus einem Gemisch von in Monomer gelöstem Peroxid mit Füllstoff und weiteren üblichen Zusätzen, während die zweite Paste aus einer Monomer/Aminlösung und Füllstoff und weiteren Zusätzen hergestellt wird. Auf diese Weise kann vom Hersteller durch intensives Verkneten und/oder Evakuieren eine besonders gute Benetzung des Füllstoffs durch die Monomerflüssigkeit erfolgen, was zur erhöhten mechanischen Festigkeit des ausgehärteten Zements beiträgt. Der Knochenzement wird in einfacher Weise durch Vermischen von äquivalenten Teilen beider Pasten kurz vor der Anwendung hergestellt und kann sofort appliziert werden.

Es ist aber auch möglich, im gesamten Monomeranteil das Amin zu lösen und mit dem gesamten Füllstoff zu einer Paste zu verarbeiten, in die dann kurz vor der Anwendung die Lösung oder Suspension des Peroxids in einem Weichmacher eingearbeitet wird.

Bei der Verwendung der Barbitursäure- oder Malonylsulfamid-Initiator-systeme werden in der flüssigen Monomerphase günstigerweise die Kupferverbindung und der Halogenidionendonator gelöst. Diese Lösung kann nun mit dem Füllstoff versetzt werden, während Barbitursäure oder Malonylsulfamid einerseits und Peroxid andererseits zusammen mit einem Weichmacher getrennt gelagert und kurz vor der Anwendung untergemischt werden. Es ist jedoch auch möglich, Barbitursäure bzw. Malonylsulfamid und Peroxid in fester Form unter den Füllstoff zu mischen und unmittelbar vor der Anwendung mit der kupfer- und halogenidhaltigen Monomerlösung anzuteigen.

Ferner ist es möglich, dem Knochenzement in an sich bekannter Weise auch Medikamente, z. B. Antibiotika zur Verhinderung von Entzündungen, zuzusetzen. Als Antibiotikum hat sich dabei besonders Gentamycin bewährt.

Die Komponenten des Knochenzements müssen in steriler Form zur Anwendung bereitgestellt werden. Soweit die einzelnen Komponenten nicht per se steril sind, können sie durch an sich bekannte Methoden, z. B. durch ionisierende Strahlen, durch Erhitzen, durch Sterilfiltration oder durch Behandlung, z. B. mit Ethylenoxid, sterilisiert werden. Dabei ist darauf zu achten, daß wirksame Initiatorkomponenten, z. B. das Peroxid, nicht zerstört werden.

Zur Kontrolle der Vermischung bei Zwei-Pasten-Systemen und zum besseren Kontrast in der

Operationssituation ist es günstig, dem Knochenzement Pigmente und/oder Farbstoffe zuzufügen. Dabei sind besonders physiologisch völlig unbedenkliche farbgebende Bestandteile geeignet, z. B. Chlorophyll oder Ultramarinblau.

Die Beispiele erläutern die Erfindung.

**Beispiel 1**

Eine Lösung von 1,4 g p-Chlor-benzoyl-peroxid in 142,5 g 2,2-Bis-[p-(γ-hydroxypropoxy)-phenyl]-propan-dimethacrylat wird mit 20,0 g pyrogener Kieselsäure und 213 g silanisiertem Quarz unter Vakuum sorgfältig zu einer homogenen Paste verknetet (Paste A).

Eine Lösung von 0,32 g N,N-Bis-hydroxyethyl-3,5-di-tert.-butyl-anilin in 142,5 g 2,2-Bis-[p-(γ-hydroxypropoxy)-phenyl]-propan-dimethacrylat wird mit 20,0 g pyrogener Kieselsäure, 140,0 g Bariumsulfat für Röntgenzwecke und 97 g fein zerteiltem, silanisiertem Quarz, sowie 160 mg Chlorophyll unter Vakuum zu einer homogenen Paste verknetet (Paste B).

Gleiche Gewichtsteile von Paste A und Paste B werden homogen vermischt. Man erhält einen plastisch verformbaren Knochenzement, der ca. 5 Minuten bei Raumtemperatur verarbeitungsfähig ist und nach ca. 10 Minuten zu einer harten Masse erstarrt ist.

Die Druckfestigkeit des Zements beträgt 170 MPa, die Biegefestigkeit 85 MPa.

**Implantierung des Knochenzements im Tierknochen**

Einem Schweineknochen wurde die obere Hälfte des Caput femuris abgesägt und in den Markraum ein Loch gebohrt. Das Innere des Markraums wurde mit einem Löffel ausgeschabt und anschließend mit Ringerlösung gespült. Eine frische Mischung der Paste A und B (1 : 1) wurde in den Markraum eingebracht. Nach dem Erhärten des Zements wurde der Femurschaft längsseitig aufgesägt und visuell inspiziert. Der Zement verbindet sich gut mit der Spongiosa und mit der Innenwand des Knochens. Ein Herausbrechen war ohne Zerstörung nicht möglich.

**Dynamische Untersuchung am Testsimulator**

Eine gebrauchte Hüftgelenkpfanne (erhöhte Reibung) wurde mit einer 1 : 1-Mischung der Pasten A und B in einen Kunststoffblock eingebettet. Nach 200 000 Zyklen mit einer Belastung von ca. 350 kp und jeweiliger Kippbewegung um 45° durch die entsprechende Gelenkkugel in einem Testsimulator wurde keinerlei Lockerungserscheinung oder Rißbildung festgestellt.

**Beispiel 2**

73,5 g Peroxidlösung von Beispiel 1 werden mit 10,0 g pyrogener Kieselsäure und 55,7 g granulierter Kieselsäure (silanisiert), nach WO 81/01366 unter Vakuum zu einer homogenen Paste verknetet (Paste C).

Weiterhin werden 71,5 g der Aminlösung des Beispiels 1 mit 10,0 g pyrogener Kieselsäure, 70,0 g Bariumsulfat, 7,0 g granulierter Kieselsäure (s. oben) und 400 mg Chlorophyll unter Vakuum zu einer homogenen Paste verknetet (Paste D).

Durch Vermischen gleicher Gewichtsteile der Pasten C und D wird ein Knochenzement erhalten, der 5,50 Minuten verarbeitungsfähig ist und nach 8,50 Minuten sich verfestigt hat. Die Druckfestigkeit beträgt 168 MPa, die Biegefestigkeit 78 MPa.

**Vergleichsversuche**

In der folgenden Tabelle sind die physikalischen Daten von zwei handelsüblichen Knochenzementen auf der Basis Methylmethacrylat/Poly(Methylmethacrylat/Methylacrylat) den Daten der erfindungsgemäßen Knochenzemente nach Beispiel 1 und Beispiel 2 gegenübergestellt.

**Tabelle**

| Zement | Max. Temperaturanstieg bei der Polymerisation (°C) | Druckfestigkeit (MPa) | Oberflächenhärte (MPa) | Wasseraufnahme (%) |
|---|---|---|---|---|
| Nach Beispiel 1 | 12 | 170 | 189 | 0,1 |
| Nach Beispiel 2 | 15 | 168 | 191 | 0,2 |
| Handelsprodukt 1 (Palacos, Kulzer) | 32 | 94 | 130 | 1,6 |
| Handelsprodukt 2 (AKZ, Howmedica) | 31 | 108 | - | - |

## Patentansprüche

1. Verwendung von difunktionellen Acrylsäure- oder Methacrylsäureestern der allgemeinen Formel

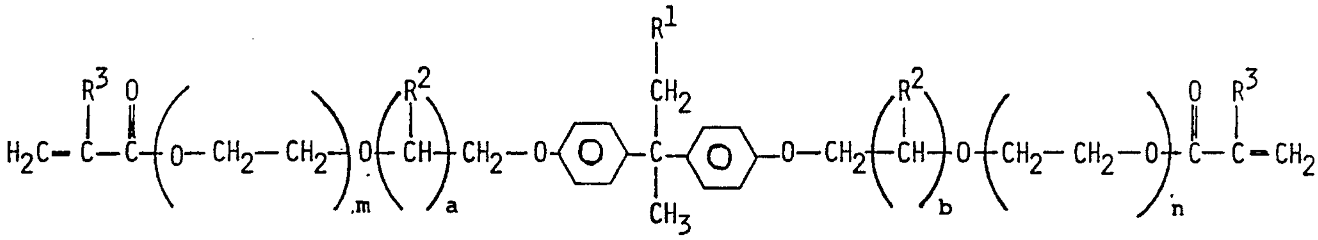

in der
a den Wert 1 oder 2 hat,
b den Wert 1 oder 2 hat,
m den Wert 0, 1 oder 2 hat,
n den Wert 0, 1 oder 2 hat,
$R^1$ H oder $CH_3$ bedeutet,
$R^2$ H, $CH_3$ oder $C_2H_5$ bedeutet und
$R^3$ H oder $CH_3$ bedeutet

zur Zementierung von Knochenin härtbaren Knochenzementen.

2. Ausführungsform nach Anspruch 1, dadurch gekennzeichnet, daß man als difunktionellen Methacrylsäureester 2,2-Bis-[p-(γ-hydroxypropoxy)-phenyl]-propan-dimethacrylat verwendet.

## Claims

1. Use of difunktional acrylic or methacrylic esters of the general formula

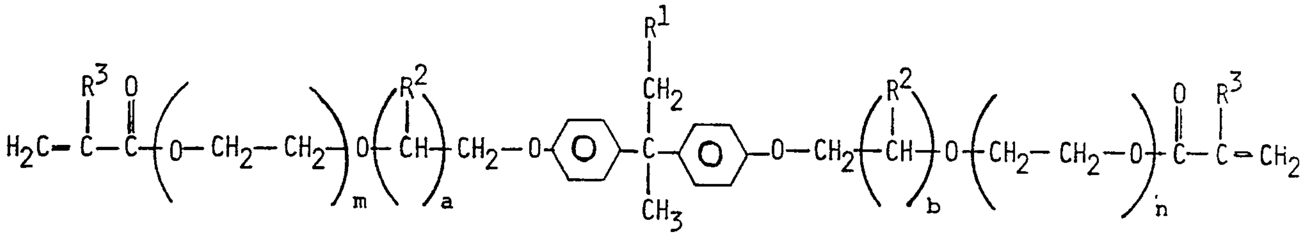

in which

a has the value 1 or 2,
b the value 1 or 2,
m the value 0, 1 or 2,
n the value 0, 1 or 2,
$R^1$ denotes H or $CH_3$,
$R^2$ denotes H, $CH_3$ or $C_2H_5$ and
$R^3$ denotes H or $CH_3$

in hardenable bone cements for cementing bones.

2. Embodyment according to claim 1, characterized in that as difunctional methacrylic ester 2,2-bis-[p-(γ-hydroxypropoxy)phenyl]-propane dimethacrylate is used.

## Revendications

1. Utilisation d'esters acryliques et métacryliques bifonctionnels de formule générale

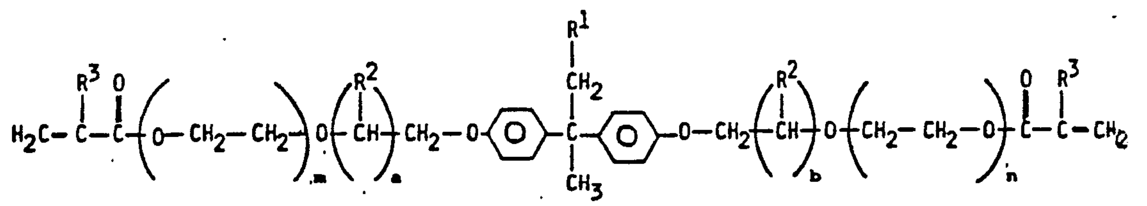

dans laquelle:

a a la valeur 1 ou 2,
b a la valeur 1 ou 2,
m a la valeur 0, 1 ou 2,
n a la valeur 0, 1 ou 2,
$R^1$ signifie H ou $CH_3$,
$R^2$ signifie H, $CH_3$ ou $C_2H_5$ et
$R^3$ signifie H ou $CH_3$,

dans des céments osseux durcissables pour la cémentation des os.

2. Mode d'exécution selon la revendication 1, caractérisé en ce qu'en tant qu'esters métacryliques bifonctionnels on utilise le 2,2-bis-[p-(γ-hydroxypropoxy)-phényl]-propane-diméthacrylate.